# FASCICULE DE BREVET EUROPEEN

(11) **EP 0 540 649 B1**
(45) Date de publication et mention de la délivrance du brevet: **09.12.1998**
(21) Numéro de dépôt: 91914325.5
(22) Date de dépôt: 24.07.1991
(51) Int. Cl.: C12P 7/02, C12P 33/06, C07J 9/00, C07J 13/00

(54) **COMPOSITION PHARMACEUTIQUE CONTENANT DES DERIVES D'HYDROXYCHOLESTEROL ET UTILISATION DE CES COMPOSITIONS POUR LA PREPARATION DE MEDICAMENTS**
HYDROXYCHOLESTEROL ENTHALTENDE PHARMAZEUTSICHE ZUSAMMENSETZUNGEN UND DEREN VERWENDUNG IN MEDIKAMENTEN
PHARMACEUTICAL COMPOSITION CONTAINING HYDROXYCHOLESTEROL DERIVATIVES AND UTILISATION OF SUCH COMPOSITIONS FOR THE PREPARATION OF DRUGS

(30) Priorité: 25.07.1990 FR 9009501
(43) Date de publication de la demande: 12.05.1993
(73) Titulaire: CENTRE NATIONAL DE LA RECHERCHE SCIENTIFIQUE, 75007 Paris (FR)
(72) Inventeur: BEHR, Patrick, F-57200 Sarreguemine (FR); KUPFERBERG, Alexandre, F-67000 Strasbourg (FR); MERSEL, Marcel, F-67000 Strasbourg (FR); PRIVAT, Alain, F-34980 S.-Clément-la-Rivière Cédex (FR)
(74) Mandataire: Warcoin, Jacques
(86) Numéro de dépôt international: FR9100608
(87) Numéro de publication internationale: WO9201803

(56) Documents cités:
- EP-A- 7 176 176
- GB-A- 1 603 864
- LIPIDS, vol. 18, no. 3, 1983; J. KANNER et al., pp. 204-210/
- BIOCHEMICAL & BIOPHYSICAL ACTA, vol. 1013, no. 3, 1989; A. KUPFERBERG et al., pp. 231-238/
- BIO ORGANIC CHEMISTRY, vol. 5, no. 1, 1976; J.I. TENG et al., pp. 99-119/
- JOURNAL OF STEROID BIOCHEMISTRY, vol. 19, no. 1A, 1983, C.J.W. BROOKS et al., pp. 189-201/
- CR Acad. Sci. Paris, 301:811-815, 1985
- Science, p. 370-371, 1968
- Cancer research, vol. 46, 1367-1373, 1986
- Cancer research, vol. 54, p. 998-1003, 1994

## Description

La présente invention concerne l'utilisation de molécules à titre d'agent cytotoxique actif sur des cellules ayant un fort potentiel de prolifération.

Les stéroïdes sont des molécules comportant un squelette cyclopentanophénanthrénique, largement répandues dans le monde vivant, et présentant une grande importance physiologique. On retrouve ainsi cette structure dans les acides biliaires, les hormones sexuelles ou corticoïdes, certaines vitamines, et les cardénolides d'origine végétale.

Un grand nombre d'entre eux possède un potentiel ou des propriétés thérapeutiques, et il est donc particulièrement utile de disposer d'une méthode simple et peu coûteuse pour en faire la synthèse. Une étape clé de cette synthèse est constituée par l'hydroxylation sélective de certaines positions du squelette.

Parmi les stéroïdes présentant un intérêt, les cardénolides constituent un groupe de stéroïdes à 23 carbones qui existent sous forme de glucosides dans différentes plantes, principalement dans la famille de la digitale, du lys et de la renoncule des champs. Beaucoup de ces glucosides possèdent une activité cardiotonique considérable et certains sont utilisés en thérapeutique, comme la digitaline, la digitoxine et la digoxine. Schématiquement, la partie aglucone de ces molécules est caractérisée par la présence d'une chaîne latérale du type "buténolide" (lactone insaturée), d'un 14-hydroxyle en position cis par rapport au méthyl 18 et d'un 3-hydoxyle. On retrouve par exemple cette structure dans la molécule de digitoxigénine de formule :

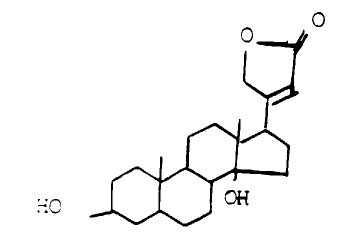

Les sucres qui forment un glucoside avec le stéroïde par une liaison établie avec l'hydroxyle en C-3 comprennent le glucose, la rhamnose, ainsi qu'un certains nombre de sucres inhabituels, tel le 2, 6-didesoxyhexose, souvent méthylé en C-3.

La manière de construire une α,β, lactone sur C17 structure cyclique nécessaire à l'activité pharmacologique de ce type de composé a été décrite notamment par Marini-Bettolo et al (Can. J. Chem, 1981, 59, 1403-1404)-. On utilise classiquement des dérivés α,βène, céto-17 comme composé de départ et l'éthoxyacétalide de lithium, le β furyl de lithium et le méthoxy-2 furyl de lithium comme réactifs. Ces procédés de synthèse ne sont pas utilisables à l'échelle industrielle, car ils présentent un certain nombre d'inconvénients. Le temps nécessaire pour obtenir le produit est relativement long et de plus, la synthèse se fait dans des conditions drastiques (solvants organiques, température élevée). D'autre part, le rendement de la réaction est faible (de l'ordre de 10%) et la purification du produit difficile, vu les nombreux produits additionnels qui apparaissent lors de la réaction. Enfin, le matériel de départ est à la fois très coûteux et très difficile à se procurer.

Un autre groupe de composés stéroïdes présentant un intérêt potentiel en thérapeutique est constitué par les oxystérols, dont la structure dérive directement de celle du cholestérol par la présence d'une ou plusieurs fonctions oxygénées portées soit par les cycles, soit par la chaîne latérale du cholestérol. On a d'abord attribué aux oxystérols un rôle dans l'inhibition de la synthèse du cholestérol.

Les oxystérols ont ensuite été étudiés en tant qu' agents antiproliférants.

Dans une revue récente, SMITH & JOHNSON (Free Radical Biology and Medicine, 1989, 7, 285-332) répertorient les cellules de mammifères atteintes in vitro par ces composés. Par une approche totalement différente, CHENG & coll (J. Chem. Res., 1977, 217, 2501-2521) ont isolé et identifié des principes actifs antitumoraux à partir de médicaments utilisés dans la pharmacopée chinoise traditionnelle. Ainsi la drogue Bombix Cum Botryte contient du 7β hydroxycholestérol qui présente une activité cytotoxique sur des cellules en culture présentant une multiplication importante, tels les lymphomes (YAC-1 et RDM4 de souris) (Cancer Biochem. Biophys. 1986, 9, 75-83), les cellules HTC (cellules tumorales de foie de rat) (Biochem. Biophys. Res. Commun., 1984, 120, 192-198), les fibroblastes de rat d'origine cardiaque (C.R. Acad. Sci. Paris, 1984, 299, 221-225) et les cellules épithéliales de foie de rat (Cell Biol. Toxicol., 1989, 5, 261-270). Par contre, les cellules dont le potentiel de division est plus faible et qui atteignent un certain degré de différenciation en culture, comme les cellules cardiaques et les hépatocytes de rat nouveau-né en culture, ne sont pas altérées par cette molécule. La cytotoxicité du 7β hydroxycholestérol se manifeste par un arrêt de la croissance cellulaire, suivi des modofications morphologiques qui précèdent le détachement et la lyse rapide des cellules.

RONG & coll (C.R. Acad. Sci. Paris, 1985, 300, 89-94) ont montré que les sels de sodium du 3,7-bis hémisuccinate de 7β hydroxycholestérol possèdent une activité antitumorale, injectés par voie intrapéritonéale à des souris porteuses en tumeur ascitique, du carcinome transplantable Krebs-II. On aussi montré, chez le rat Sprague-Dawley femelle, que le (22 R)-cholest-5-ène 3β, 7β 22-triol réduisait efficacement le développement de tumeurs induites par du 7,12-diméthylbenz (α) anthracène (Iversen et al. Virchows Archiv B, 1986, 51, 313-320).

Dans Biochem. Biophys. Acta (vol. 1013, 1989, p. 231-238), l'effet du 7 β hydroxycholestérol sur des cultures d'astrocytes spontanément transformées, obtenues par passage successif, a été étudié.

Il est généralement admis, que l'effet cytotoxique des oxystérols résulte de la superposition de plusieurs phénomènes, dont les plus importants sont l'inhibition de la synthèse du cholestérol par inhibition d'une enzyme clé (l'HMGR), et une déstabilisation des membranes plasmiques. Dans ce contexte, et dans une perspective thérapeutique à long terme, les inventeurs ont étudié les effets et le mécanisme d'action du 7β hydroxycholestérol sur des lignées spontanément transformées obtenues à partir de cultures primaires d'astrocytes de rat nouveau-né (cellules normales). Dans le système nerveux central (SNC), les astrocytes (cellules gliales) ont gardé la potentialité de se multiplier, phénomène qui se manifeste dans certains états pathologiques comme la gliose réactionnelle (inflammation) et le formation de glioblastomes (néoplasie).

Or, une publication récente montre que le 7β hydroxycholestérol est métabolisé dans les astrocytes transformés, en ester d'acides gras.

La voie de synthèse chimique classique utilisée pour obtenir des 7β hydroxycholestéryl ester fait appel au 7 céto cholestérol comme produit de départ.

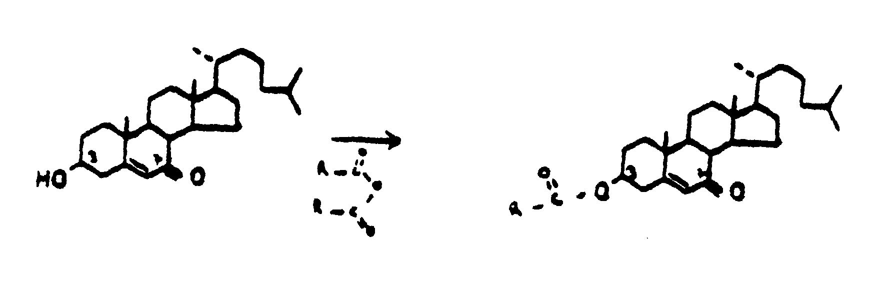

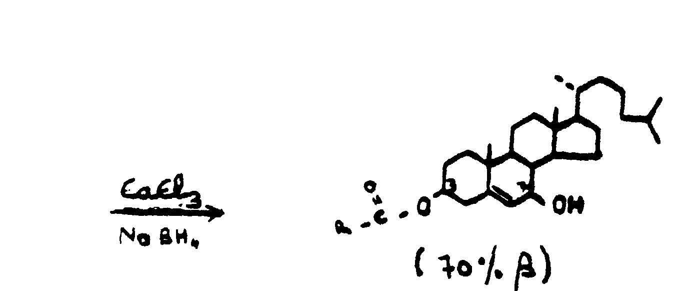

Après estérification du OH en 3, on opère une réduction de la fonction par NabH₄. On obtient les deux isomères α et β avec un rendement de 70% pour l'isomère β. Ce procédé est difficilement transposable à l'échelon industriel étant donné sa durée.

Les composés selon la présente invention peuvent être obtenus par un procédé doux d'hydroxylation d'un radical méthylène acyclique ou cyclique en position allylique, caractérisé en ce que l'on fait réagir sur ce radical méthylène, une péroxydase, des ions iodure et du péroxyde d'hydrogène (H₂O₂) pour obtenir un composé hydroxylé en α d'une double liaison.

La péroxydase utilisée est de préférence la lactopéroxydase (LPO), les ions iodures proviennent en général de KI et le péroxyde d'hydrogène peut être généré in situ.

Contrairement aux méthodes d'hydroxylation classiques, ce procédé ne fait pas intervenir de réducteur puissant.

Le radical OH est fourni par la décomposition de H₂O₂, catalysée par la lactopéroxydase, enzyme disponible dans le commerce à un coût modéré.

La réaction peut être menée à des températures comprises entre 20°C et 40°C, et respectera des fonctions présentes sur la molécule.

La présente invention concerne donc un procédé d'hydroxylation d'un méthylène acyclique ou cyclique en position allylique, caractérisé en ce que le composé de départ comporte au moins une fonction ester non protégée, et en ce que cette fonction ester est retrouvée intacte dans le composé après hydroxylation.

La présente invention concerne également un procédé d'hydroxylation d'un méthylène acyclique ou cyclique en position allylique, dans lequel le composé de départ comporte une fonction éther.

Le radical méthylène allylique est de préférence :
- un carbone secondaire (1) fixé sur le schéma la suivant : 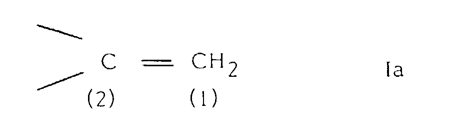 dans lequel le carbone (2) est relié de préférence à un hydrogène ou à d'autres carbones.
   A titre d'exemple de composé de ce type, on peut citer des dérivés : 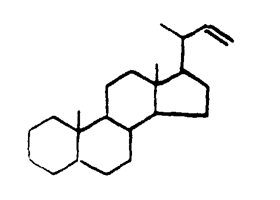 l'hydroxylation étant effectuée sur le carbone distal de la double liaison avec migration de cette dernière. Ce type de composé pouvant comporter d'autres substituants, sous réserve qu'il n'y ait pas d'autres carbones méthyléniques allyliques.
- ou bien un carbone secondaire (1) fixé selon le schéma Ib suivant : 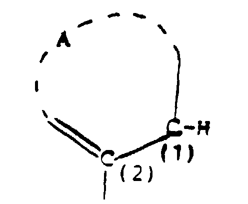 dans lequel (A) est une structure cyclique qui est de préférence de type prégnane ; par exemple le carbone méthylénique étant en position 7 du système cyclique Δ 5 pregnène.
   A titre d'exemple, on peut citer : 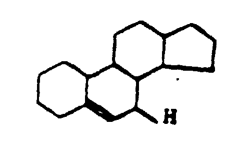 l'hydroxylation étant effectué sur une position α à la double liaison (6-7). Ce type de composé pouvant comporter d'autres substituants sous réserve qu'il n'y ait pas d'autres carbones méthyléniques-allyliques.

Dans ce procédé d'hydroxylation, on fait réagir la LPO, KI et H₂O₂ sur de l'acétate 3, Δ 22-23 norcholène de formule : 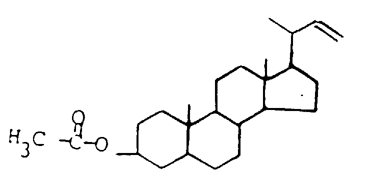 et on obtient de l'acétate 3, ol 23, Δ 17-20 norcholène de formule : 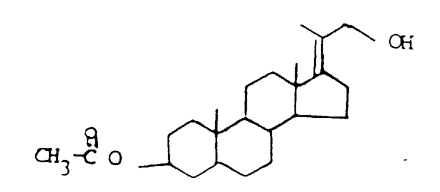 en une seule étape.

L'acétate 3, Δ 22-23 norcholène est obtenu à partir de l'acide ol 3, cholanique. Ce composé, dont le prix d'achat est peu élevé, est facilement transformé en ol 3, Δ 22-23 norcholène selon la méthode de Vaida & al (Tétrahédon Lett., 1968, 50, 5173-5174) modifiée.

L'acétate 3, ol 23, Δ17-20 norcholène permet ensuite, par une série de réactions schématisées ci-dessous, l'obtention d'un cardénolide aglucone

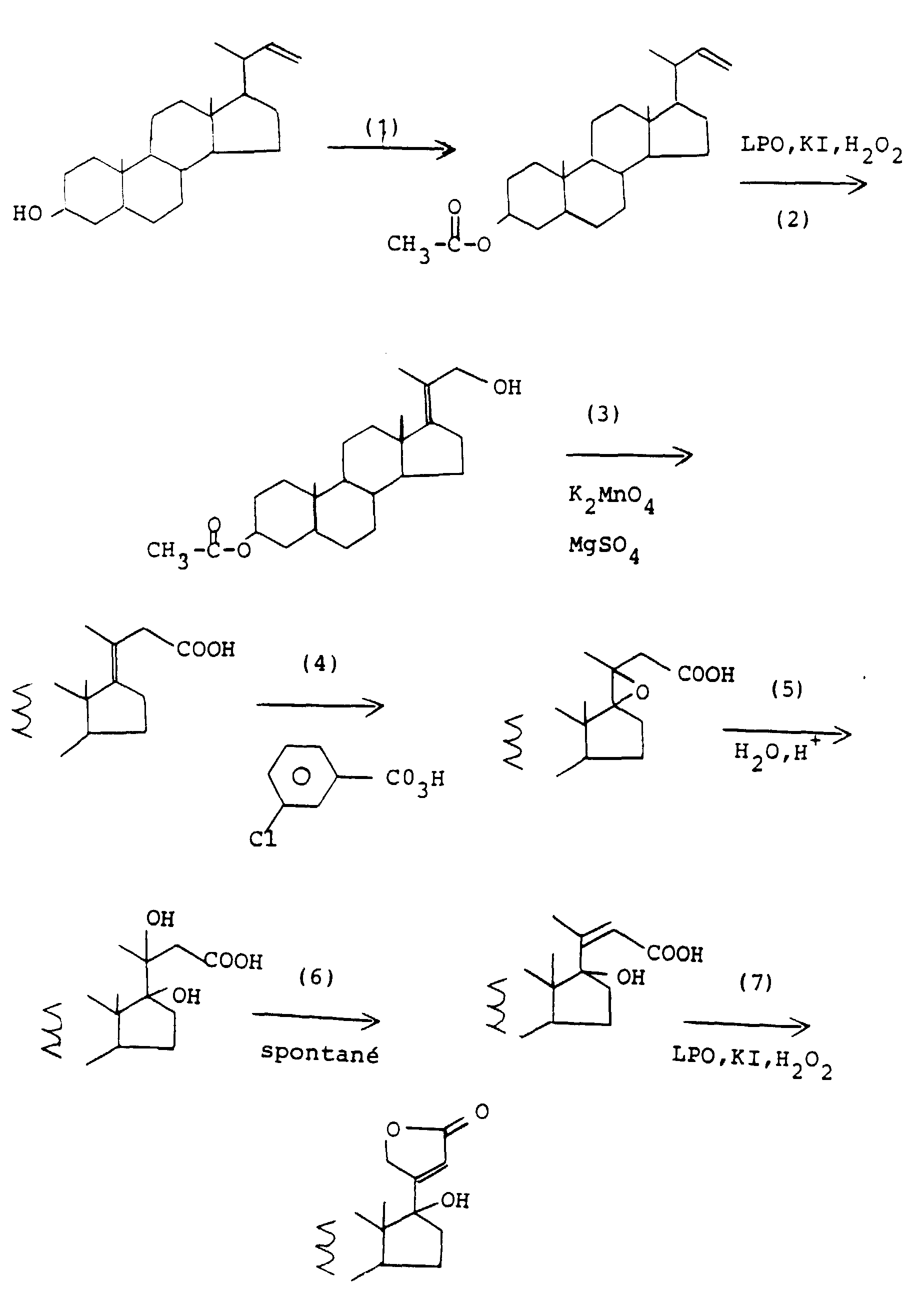

Cette méthode est simple, demande peu d'énergie, sa durée est courte et son prix de revient bas, vu le coût faible des réactifs. En outre, la réaction présente un bon rendement et permet la préparation du produit à l'échelle semi-macro, avec peu de produits additionnels.

En effet, du fait de sa spécificité, l'utilisation d'un enzyme comme catalyseur favorise l'obtention d'un produit majeur.

Cette voie de synthèse permet l'activation des carbones 17, 20 et 23. De ce fait, d'autres transformations peuvent être effectuées avant d'obtenir le produit final. Par cette méthode, des produits possédant une plus grande activité pharmacologique et une moindre toxicité peuvent être obtenus.

Selon un autre aspect, le procédé d'hydroxylation est caractérisé en ce que l'on fait réagir une péroxydase, des ions iodure, et le péroxyde d'hydrogène, sur un mono ester en C3 de cholestérol de formule : 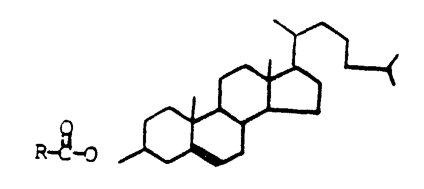 dans laquelle R représente une chaîne carbonique correspondant à un acide gras,
et en ce qu'on obtient un ester de 7 hydroxycholestérol de formule : 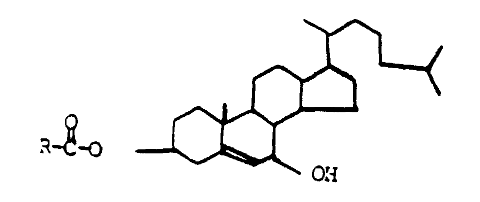 sous forme d'un mélange d'esters de 7α et 7β hydroxycholestérol.

Les acides gras sont des constituants universels de tous les composés lipidiques, et sont en majorité à nombres pairs d'atomes de carbone (au moins 4) et à chaîne linéaire. Les acides gras naturels peuvent être saturés ou insaturés, en général ils présentent alors de 2 à 6 doubles liaisons ; il existe également des acides gras naturels à triple liaison.

Ces acides gras peuvent estérifier la fonction alcool en 3 de la molécule de cholestérol. Contrairement aux monoesters du 7 céto cholestérol utilisés pour la préparation des dérivés hydroxy, les monoesters du cholestérol sont facilement disponibles à un coût peu élevé.

La réaction se fait en une seule étape, à une température comprise entre 20°C et 37°C, dans un solvant organique lipophile.

On obtient un mélange d'isomères α et β de 7 hydroxycholestérylesters à des proportions de 50% et 50% respectivement. Le rendement peut être optimalisé par l'adaptation des solvants, de la concentration des réactifs et de la température de réaction, en fonction du type d'isomère désiré.

La présente invention concerne également les esters d'acide gras en C₃ de β hydroxycholestérol substitués ou non qui peuvent notamment être obtenus par le procédé selon l'invention. Les acides gras comportent de préférence de 6 à 24 atomes de carbone et peuvent comporter une ou plusieurs insaturations

Dans un aspect préféré de l'invention, le produit de départ est choisi parmi le groupe des esters de cholestérol comprenant le palmitate, l'oléate, l'héxénoate, le décénoate et l'arachidonate de cholestérol.

L'acide palmitique est un acide gras aliphatique saturé de formule générale C₁₆H₃₂O₂.

Les acides oléïque, hexénoïque et décénoïque sont des acides gras monoéthéniques ayant respectivement pour formule brute C₁₈H₃₄O₂, C₆H₁₀O₂ et C₁₀H₁₈O₂. L'acide arachidonique possède quatre doubles liaisons et répond à la formule C₂₀H₃₂O₂.

Pour les applications cytotoxiques et antitumorales, dans le procédé d'hydroxylation selon l'invention d'un monoester en C3 du cholestérol, on isole du mélange réactionnel l'isomère 7 β de l'hydroxycholestéryl ester obtenu, ledit isomère appartenant au groupe comprenant notamment les esters d'acide palmitique, oléique, hexénoïque, décènoïque, et arachidonique.

Selon un autre de ses aspects, le procédé d'hydroxylation consiste à faire réagir une péroxydase, des ions iodure et le péroxyde d'hydrogène sur un éther en C3 de cholestérol de formule : 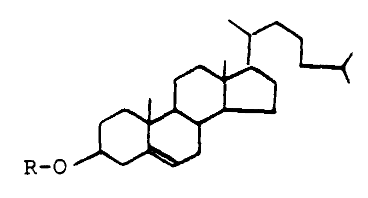 dans laquelle R représente une chaîne carbonée correspondant à un acide gras, pour obtenir un éther de 7 hydroxycholestérol de formule :

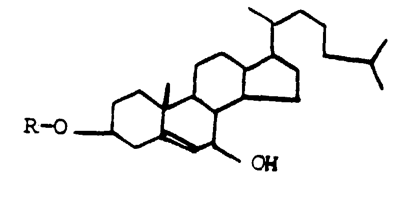

De préférence, on isole du mélange réactionnel l'isomère 7β de l'hydroxycholestéryl éther obtenu, ledit isomère appartenant en particulier au groupe comprenant les éthers d'acide palmitique, oléique, hexénoïque, décénoïque et arachidonique.

Ces composés peuvent également être synthétisés à partir du 7-céto-cholestérol et, du chlorure d'alcènyle correspondant à l'acide gras, par exemple CH₃-(CH₂)₇-CH=CH-(CH₂)₇-CH₂Cl pour l'acide oléique. On procède ensuite à une réduction par NaBH₄.

La formation d'un éther entre la chaîne carbonée de l'acide gras et la molécule de stéroïde, au lieu d'une fonction ester, donne une molécule plus stable qui peut résister à l'hydrolyse, notamment par les enzymes hépatiques, et peut donc être injectée de façon périphérique.

C'est pourquoi la présente invention a pour objet une composition pharmaceutique, caractérisée en ce qu'elle comporte au moins un éther en C-3-OH du 7β hydroxycholestérol choisi dans le groupe comprenant les éthers d'acide palmitique, oléique, hexénoïque, décénoïque et arachidonique, et un support acceptable pour son administration.

Une telle composition est utile en tant qu'agent cytotoxique sur des cellules possédant un fort potentiel de prolifération.

La présente invention se rapporte également à une composition pharmaceutique, caractérisée en ce qu'elle comporte au moins un mono ester d'acide gras en C3 de 7 β hydroxycholestérol de préférence choisi dans le groupe comprenant le palmitate, l'oléate, l'héxénoate, le décénoate, et l'arachidonate, sous forme de liposomes et/ou associé avec des phospholipides et/ou des gangliosides et un support acceptable pour son administration.

En particulier, la présente invention concerne l'utilisation d'un mono ester en C₃ de 7β hydroxycholestérol et d'un acide gras tel que défini plus haut, pour la préparation d'une composition pharmaceutique destinée au traitement de cellules possédant un fort potentiel de prolifération. En effet, cette composition est utile en tant qu'agent cytotoxique.

Selon un autre aspect de l'invention, on utilise un ester en C7 du 7β hydroxylcholestérylester décrit précédemment. Dans le cas où des composés plus hydrophiles sont recherchés, une molécule particulièrement intéressante est représentée par le 7-phosphoénol-pyruvate-3 oléate cholestéryl.

Les composés liposolubles du 7β hydroxycholestérol peuvent être mis sous forme de liposomes ou d'association avec des phospholipides tels que ceux utilisés dans la préparation des liposomes, phosphatidylcholine notamment. Ils peuvent également être associés, au sein des liposomes, à des gangliosides de type GM1 et GT1b (trisialoganglioside). Cette association entre des gangliosides et des esters en C3 du 7 β hydroxycholestérol permet d'obtenir un vecteur de plus petite taille et plus hydrophile.

Ces liposomes vont être administrés par voie parentérale avec un support acceptable.

Ces compositions présentent une grande affinité pour le tissu nerveux, riche en lipides.

Les compositions renfermant un éther ou un ester de β hydroxycholestérol et d'acide gras, incorporé dans des liposomes contenant de la phosphatidylcholine et par exemple du monosialoganglioside (GM1) sont ainsi particulièrement utiles comme antitumoral sur des tumeurs du système nerveux de type glioblastomes intracérébraux, ou comme agent de traitement de glioses réactionnelles.

La présente invention se rapporte donc à la préparation d' une composition pharmaceutique comprenant au moins un monoester en C3 du 7β hydroxycholestérol sous forme de liposomes, et utile en tant qu'agent antitumoral. Selon un aspect de l'invention, cette composition pharmaceutique est caractérisée en ce qu'elle est utile sur des tumeurs du système nerveux, en particulier des glioblastomes. L'injection de liposomes contenant de la phosphatidylcholine et certains esters du 7β hydroxycholestérol fait régresser de façon significative des glioblastomes sous-cutanés induits chez le rat.

Selon un autre aspect de l'invention, la composition pharmaceutique comprenant un monoester du 7β hydroxycholestérol sous forme de liposomes, est utilisée en tant qu'agent anti-inflammatoire, du système nerveux, en particulier dans des glioses réactionnelles.

Les exemples qui suivent sont destinés à illustrer l'invention sans aucunement en limiter la portée.

On se référera aux figures suivantes ci-annexées :
- **FIGURE 1 :**: Gliose réactionnelle ; traitement au cholestéryl-oléate.
- **FIGURE 2 :**: Gliose réactionnelle ; traitement au 7β -OH-CH (7β hydroxycholestérol).
- **FIGURE 3 :**: Gliose réactionnelle ; traitement au 7β -OH-cholestéryl-3-oléate.
- **FIGURE 4 :**: Tumeur sous-cutanée induite chez le rat par l'inoculation de cellules C6.
- **FIGURE 5 :**: Traitement des tumeurs sous-cutanées au cholestéryl-oléate.
- **FIGURE 6 :**: Traitement des tumeurs sous-cutanées au 7β -OH-choles-téryl-3-oléate.
- **FIGURE 7 :**: Microscopie optique des glioblastomes après coloration au crésyl violet.

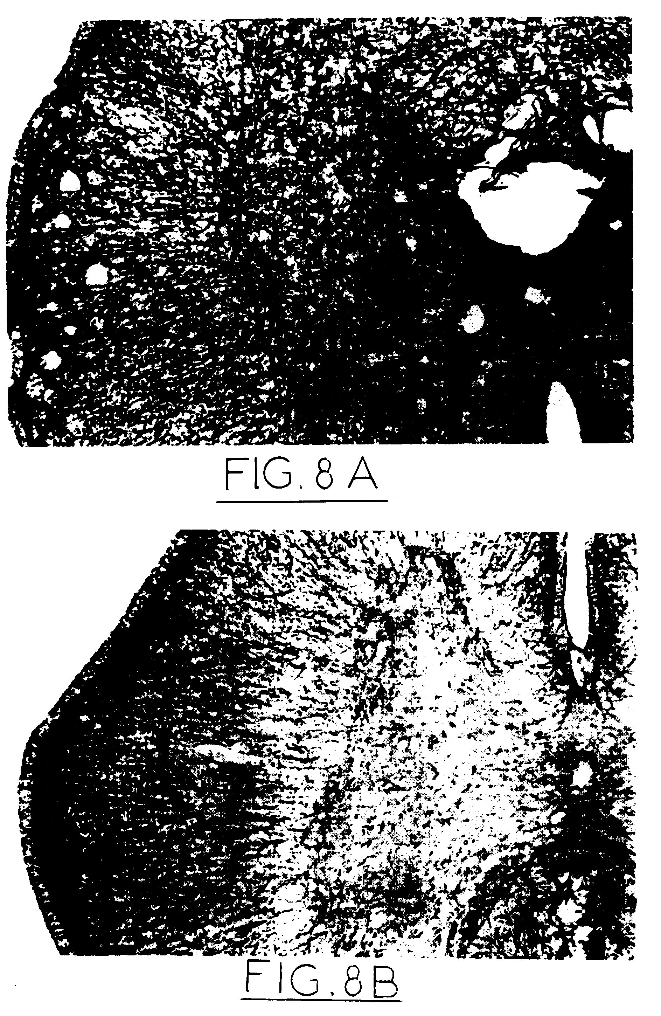

- **FIGURE 8 :**: Détection immunocytochimique de la protéine gliofibrillaire acide (GFAP) dans une moelle épinière après section.
8A, rat témoin.
8B, rat traité par des liposomes contenant du 7 β hydroxycholestéryl-3-oléate, de la phosphatidylcholine et du GM1.

### EXEMPLE 1 : MATERIELS ET REACTIFS

- Couches minces de silice (TLC) sans ou avec indicateur de fluorescence F254 (Merck, RFA)
- Colonne HPLC : colonne de silice (5µ, 3,9 mm x 15 cm) (Waters, USA)
- Appareillage HPLC (Waters Associated, USA)
- Chromatographie en phase gazeuse (colonne capillaire du type SE30) couplée à la spectrométrie de masse (LKB 9000, Suède)
- RMN 200 ou 400 MHz (Bruker, RFA)
- Microscopie électronique (Philips EM300, Pays-Bas)
- Sonicateur (Sonimasse 520 ou Soniprep 150, France)
- Microscopie à constrate de phase ou à lumière (Nikon, Japon)
- Lampe à UV (Bioblock, France)
- Lactoperoxydase (EC 1.11.1.7, 60 UI/mg protéines, Sigma, USA)
- H₂O₂ (Prolabo RP, Normapur, France)
- Anticorps de lapin anti GFAP (Glial Fibrillary Acid Protein) (Dakopatts, Danemark)
- Anticorps secondaires couplés à la peroxydase (Biosys, France)
- Les clones de cellules (glioblastomes) ont été fournis par le Prof. Benda (Science, 1968, 161, 370-371).
- Les autres réactifs et solvants sont du type Analar.

### HPLC

Les produits extraits des TLC sont élués à 500 P.S.I. dans du n-heptane-isopropanol (99:1 v/v) (débit de 1,5 ml/min) et détectés par un spectrophotomètre à 220 nm. Après évaporation et dilution dans le solvant approprié ou/et silylation (Kupferberg et al., Biochim. Biophys. Acta., 1989, 1013, 231-238). Les échantillons récoltés sont soumis à l'analyse par RMN et/ou par spectrométrie de masse couplée à la chromatographie en phase gazeuse (GC-MS)

### GC-MS :

- GC : Colonne : 25 m, diamètre interne 0,32 mm (SE30)
   Gaz vecteur : hélium 15 P.S.I.
   Programme de température : 100°C à 300°C (3°C/min)
   Injecteur du type ROS (280°C)
- SM : Température de séparation : 250°C
   Température de la source : 250°C
   Energie électronique : 70eV
   Potentiel d'accélération : 3500 V

### RMN :

Les composés à analyser ont été dissous dans du CDCl₃.

### EXEMPLE 2 : SYNTHESE DU DIOL 3, 23, Δ 17-20 NORCHOLENE (Ic1)

4 mg d'ol 3, Δ 22-23 norcholène sont prelevés à partir d'une solution de chloroforme (CHCl₃) et le solvant évaporé dans un ballon sous atmosphère d'azote, 0,9 ml de diméthylsulfoxide est ajouté au résidu et le tout est chauffé à 30°C pendant quelques minutes. A cette solution sont ajoutés dans l'ordre :
- 15 ml de tampon phosphate (50 mM, pH 6,0, Pi)
- 1 ml de Pi contenant 15 mg de KI
- 450 µl de Pi contenant 450 µg de LPO.

La réaction est initiée en ajoutant 300µl de H₂O₂ à 1,3 % toutes les 15 sec et ceci 10 fois de suite. Le mélange du milieu réactionnel est réalisé par un barreau magnétique. Le milieu réactionnel est ensuite extrait successivement avec 20 ml, 5 ml et 5 ml de CHCl₃. Les phases chloroformiques rassemblées sont séparées avec 10 ml H₂O₂. La phase organique est alors filtrée sur du sulfate de sodium anhydre et évaporée. La chromatographie des produits se fait sur TLC avec trois élutions successives dans le système de solvants (cyclo-hexane-acétate d'étyle, 2:1 v/v) et les produits sont détectés soit à 254 nm, soit par le réactif de Macala et coll. (J. Lipid Res., 1983, 24, 1243-1250). Les produits sont extraits de la silice par du CHCl₃ (1:1 v/v) évaporés et soumis à l'analyse.

### RESULTATS :

Le spectre RMN montre l'apparition de deux pics à 0,67 et 0,73 ppm, auxquels nous attribuons la résonance du méthyle-18 de deux produits isomères géométriques. Nos raisons sont les suivantes :
- le méthyl-21 apparaît comme deux "singlets" à la place du "doublet" observé pour le produit de départ (ol 3, Δ22-23 norcholène) ;
- des protons vinyliques ne sont pas détectés ;
- au voisinage du proton-3, deux protons additionnels sont détectés. Leur forme et distribution complexes suggèrent des effets à longue distance sur le déplacement chimique (chemical shift) de ces derniers.

L'apparition de deux protons dans un champ magnétique bas et la complexité de leur spectre indiquent la présence d'une double liaison ( Δ 17-20).

La structure probable du composé est donc du dihydroxy-3, 23, Δ 17-20 norcholène sous forme de deux isomères géométriques. La RMN du produit Ic1 acétylé confirme notre analyse.

Le rendement de cette synthèse enzymatique est de 90 %.

### EXEMPLE 3 : SYNTHESE DE L'ACETATE 3, Δ 22-23 NORCHOLENE

4 mg d'ol 3, Δ 22-23 norcholène sont dissous dans 2 ml de CHCl₃ et 100µl de pyridine anhydre et 300 µl d'anhydre acétique sont ajoutés. Le milieur réactionnel est continuellement mélangé à température ambiante pendant 24 h. Après évaporation sous azote les produits sont fractionnés et détectés comme décrit dans l'exemple précédent.

### EXEMPLE 4 : SYNTHESE DE L'ACETATE 3, ol 23, Δ 17-20 NORCHOLENE (IC)

La préparation des réactifs et la synthèse se font à 30°C. 4 mg d'acétate 3, Δ 22-23 norcholène sont prélevés à partir d'une solution chloroformique et le CHCl₃ évaporé dans un ballon sous atmosphère d'azote ; 1 ml de diméthyl-sulfoxide est ajouté au résidu et sont ajoutés ensuite dans l'ordre :
- 11 ml de Pi (goutte à goutte)
- 500 µl de Pi contenant 7,5 mg de KI
- 1,7 ml d'une solution composée de 1 ml d'H₂O₂ à 1,3 % et de 0,7 ml contenant 200 µg de LPO.

Au bout de 1 h sont ajoutés quelques cristaux de I₂ sublimé (2 mg environ) et au bout de 6 heures sont ajoutés :
- 500 µl de Pi contenant 7,5 mg de KI
- 1 ml d'H₂O₂ à 1,3 %
- 0,2 ml de Pi contenant 200 4g de LPO.

Cette dernière opération est répétée après 24 h. L'extraction, le fractionnement et la détection des produits se font selon l'exemple 2.

### RESULTATS :

La silylation de Ic et la GC-SM montrent la masse du composé supposé moins un fragment de 42 de masse (M-42); la masse 42 correspond à un fragment de l'acétyle, clivé lors de l'impact électronique. Ce résultat démontre la présence des groupements acétyle et hydroxyle.

La RMN, qui montre la disposition du "doublet" (méthyle 21) résonnant à 1,02 et 1,03 ppm et des "quadruplet" et "quintuplet" résonnant à 4,83 et 5,82 ppm respectivement (protons 23 et 22), plaide en faveur de la migration de la double liaison 22-23 entre les carbones tertiaires (Δ 17-20). L'apparition d'un "triplet" à 3,12 ppm, d'un autre centré à 3,65 ppm et dédoublé ainsi que d'un "doublet" centré à 3,90 ppm plaide en faveur du groupement (-CH₂[C22]-CH₂OH[C23] à proximité de la double liaison (Δ 17-20).

Le rendement de cette synthèse enzymatique est de 70 %.

### EXEMPLE 5 : SYNTHESE DU 7β -OH-CHOLESTERYL-PALMITATE (C16:0)

40 µg de cholestéryl-palmitate sont prélevés à partir d'une solution chloroformique et le CHCl₃ évaporé sous azote ; 100 µl de diméthylsulfoxide et 200 µl d'éther [(CH₃CH₂)O] sont ajoutés au résidu et la solution chauffée successivement à 100°C pendant 20 min et à 120°C pendant 15 min dans une enceinte hermétique. La solution est équilibrée à 37°C et la réaction se fait à cette température. 670 µl de Pi sont ajoutés (goutte à goutte) ainsi que 200 µl de Pi contenant 200 µl de LPO, 50 µl de Pi contenant 750 µg de KI et 10 µl de Pi à 1,3 % de H₂O₂ toutes les 15 sec et ceci 10 fois. Après 30 min d'incubation, le cycle (LPO, KI, H₂O₂) est répété une seconde fois et au bout de 1 h les produits sont extraits avec 4 ml de CHCl₃, les phases chloroformiques sont rassemblées et rincées avec 4 ml d'H₂O deux fois. Après évaporation, les produits sont fractionnés sur TLC avec trois élutions successives dans le système de solvants hexane-éther (8:2 v/v). Les produits sont ensuite détectés et extraits de la silice comme décrit dans l'exemple 2.

### EXEMPLE 6 : SYNTHESE DU 7β -OH-CHOLESTERYL-OLEATE (MET C18:1)

40 µg de cholestéryl-oléate sont prelevés à partir d'une solution chloroformique et le CHCl₃ évaporé sous azote ; 100 µl de diméthylsulfoxide et 100 µl de benzène sont ajoutés et le milieu soniqué pendant 30 sec à 180 µ d'amplitude à température ambiante. 200 µl de Pi contenant 200 µg de LPO ainsi que 50 µl de Pi contenant 75 µg de KI sont rapidement ajoutés. La réaction est initiée avec l'addition de 10 µl de Pi contenant 1,3 % de H₂O₂ dix fois toutes les 15 sec. Le cycle LPO/KI/H₂O₂ est répété après 30 min et au bout de 1 h les produits sont extraits, chromatographiés et identifiés comme décrits aux exemples 5 et 2 respectivement.

### RESULTATS DE SYNTHESE ENZYMATIQUE DU 7β -OH-CH₃ PALMITATE OU OLEATE :

Les esters en C-3-OH (C16:0 et C18:1) du 7β -OH-CH ont été synthétisés par voie chimique selon le schéma Il et ont servi de témoins à l'identification de ces mêmes molécules synthétisées enzymatiquement (LPO) par GC-SM, vu les micro-quantités de matériel utilisées.

L'analyse en GC-SM montre clairement l'obtention des 7β -OH-cholestéryl-palmitate et oléate respectivement. Le rendement de la réaction est de 10 % et celui des isomères α et β, 50% et 50 % respectivement.

### EXEMPLE 7 : GLIOSES REACTIONNELLES IN VIVO ET TRAITEMENT

### Préparation des liposomes (phosphatidylcholine,PC,/stéroïdes)

PC et dérivés stéroïdes sont dissous dans du CHCL₃-méthanol (2:1 v/v), le solvant évaporé et 1 ml de KCL (0,15 M) ajouté. La suspension est soniquée dans un bain de glace (5x1 min à 30 sec d'intervalle, 18 µm d'amplitude), centrifugée 13.000g pendant 20 min, le surnageant prélevé et concentré 5 fois sous azote.

### CERVEAU

Des rats agés de 6 jours sont anesthésiés par une injection intrapéritonéale d'imalgène 500 (2 mg/10 g) et de valium (0,05 ml/10 g) et placés ensuite dans un appareil stéréotaxique afin de subir une lésion électrolytique au niveau du striatum. Pour cela, une sonde dentaire isolée, sauf à la pointe, est descendue jusqu'au niveau du striatum selon les coordonnées stéréotaxiques par rapport au lambda. A ce niveau, un courant de 2mA est appliqué pendant 10 sec ;
2 µl de suspension de liposomes contenant 20 µg de PC et 2 µg de stérols ou d'oxystérols divers sont injectés dans le site de lésion.

### MOELLE EPINIERE

Après section complète de la moelle épinière au niveau de T8 chez des rats de 250 g (Sprague, Dawley, EFFA, Credo), 5 µl d'une suspension de liposomes contenant 500 µg de PC et 50 µg d'esters du 7β -OH-CH (C18:0 ou C18:1) sont injectés à 0,5 mm de la section, en aval.

### DETECTION DES ASTROCYTES REACTIFS :

La GFAP, marqueur structural spécifique des astrocytes, est détectée sur des coupes paraffines par le système d'anticorps, anti-anticorps à la GFAP couplé à la peroxydase. L'addition de 4-chloro-naphtol et d'H₂O₂ révèle la présence de GFAP.

### EFFET DES ESTERS DU 7β -OH-CH SUR DES GLIOSES REACTIONNELLES ET DES GLIOBLASTOMES SOUS-CUTANES C-3-OH IN VIVO

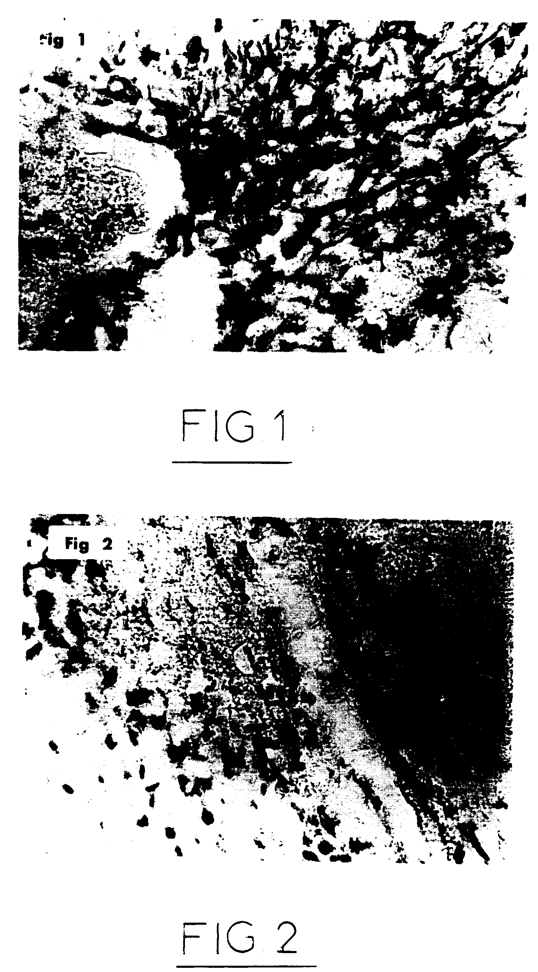

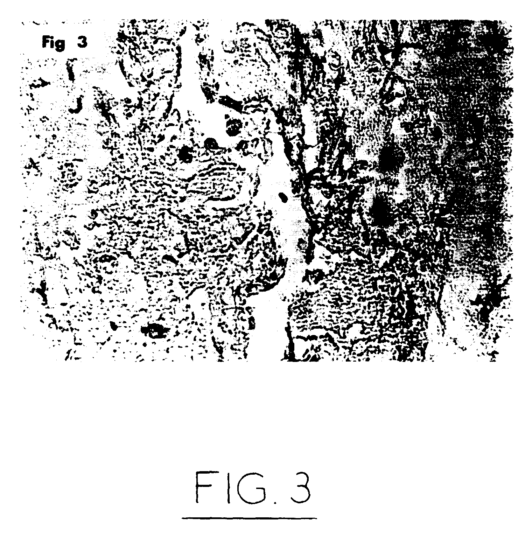

L'injection de liposomes contenant du 7β -OH-CH (C18:1) montre une forte atténuation de la gliose réactionnelle dans le SNC (fig. 3) par rapport à l'injection de 7β -OH-CHj (fig. 2) ou de CH (C18:1) (fig. 1). De même, l'injection de liposomes contenant soit du PC, soit du PC/CH, soit du PC/CH (C16:0), et, soit du PC/7β -OH-CH (C18:0) s'est montrée inefficace. L'injection de liposomes PC/7β -OH-CH (C18-1) à côté du site lésion de moelle épinière atténue aussi la gliose réactionnelle secondaire.

### EXEMPLE 8 : INDUCTION DE TUMEURS SOUS-CUTANEES IN VIVO ET TRAITEMENT

Les cellules C6 (passage 55-57) sont injectées par voie sous-cutanée au niveau des épaules à raison de 10⁶ cellules/200 µl de NaCI 9 ‰ dans des rats (26 g) de 3 semaines selon Ledig et coll. (Revue de l'Alcoolisme, 1986, 31, 1-11) ; lorsque les tumeurs se développent (2 semaines après l'inoculation des cellules C6), 50 µl d'une suspension de liposomes contenant 1 mg de PC et 100 µg de cholestéryl-oléate ou de 7β -OH-cholestéryl-oléate sont injectés dans les tumeurs. Les animaux sont photographiés 48 h après.

### RESULTAT SUR LES GLIOBLASTOMES SOUS-CUTANES

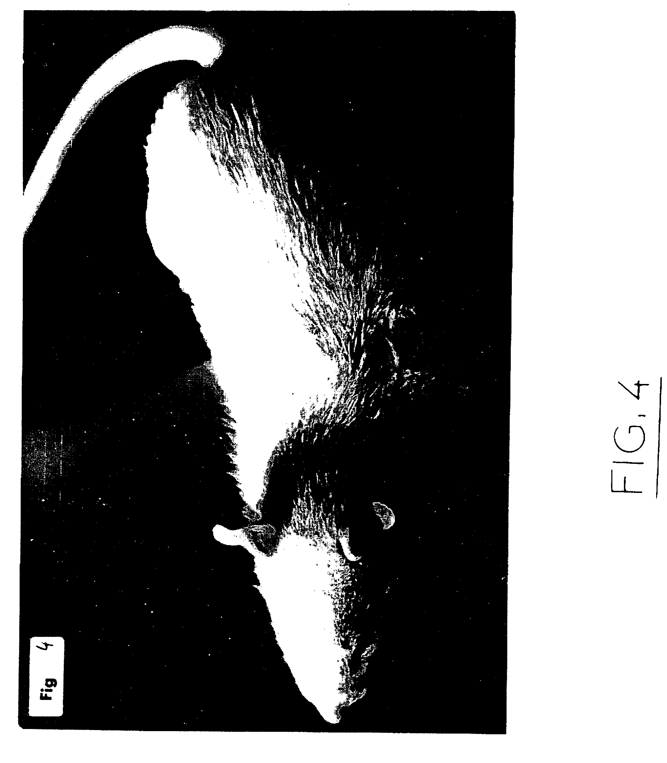

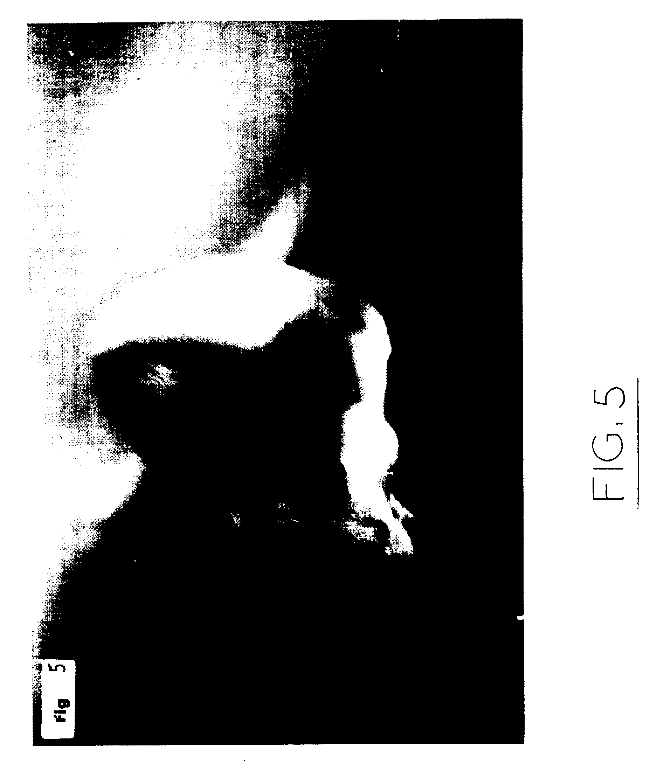

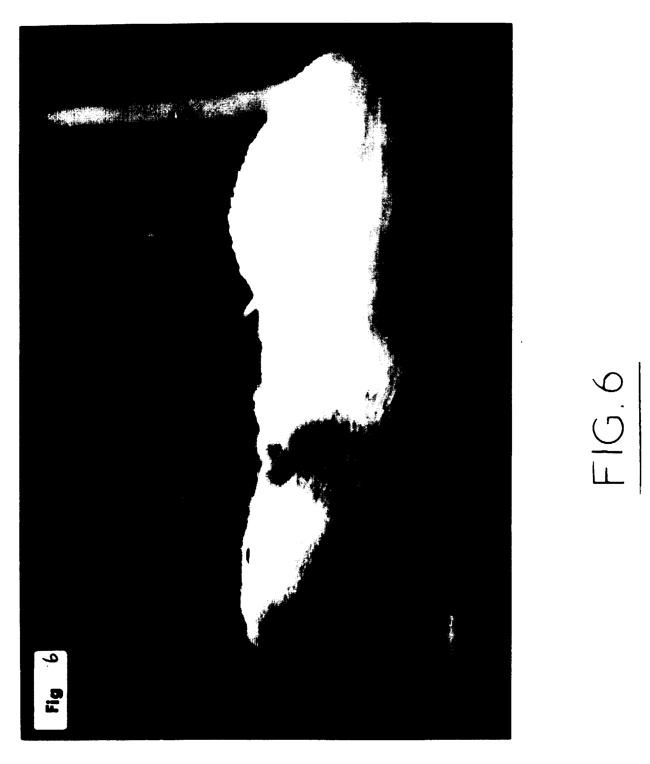

La figure 4 montre une tumeur développée chez un rat mâle par inoculation de cellules C6 ; alors que l'injection de liposomes contenant PC/CH (C18:1) n'a pratiquement aucun effet (fig. 5), celle de liposomes contenant PC/7β -OH-CH (C18:1) fait regresser la tumeur d'une façon significative (fig. 6).

### EXEMPLE 9 : ACTIVITE SUR DES GLIOBLASTOMES IN VIVO DE L'ESTER C18:1

### INDUCTION DES TUMEURS DANS LE CERVEAU

Le modèle "in vivo" proposé par Galli et coll (J. Neuro-Oncology, 7, 299-304. 1989.) a été adopté avec quelques modifications au niveau du nombre de cellules du type C6 injectées et de l'âge des rats. Des rats (Wistar mâle) âgés de 6 jours sont anesthésiés et placés dans un appareil stéréotaxique, la peau du crâne est ouverte et la boîte crânienne percée aux coordonnées adéquates (4,5 AP, 3,0 ML, 2,0 mm DV par rapport au lamda, repère 0) à l'aide d'une fraise dentaire ; 200x10³ C6 dans 5 ml de PBS sont ensuite injectées en 4 minutes et ensuite la peau suturée. 72 heures plus tard, les rats sont opérés à nouveau et traités par des liposomes contenant les 7β -hydroxycholestéryl-3-esters.

### PREPARATION DES LIPOSOMES

Les liposomes sont préparés selon la méthode de Manyama et coll. De la phosphatidylcholine, du GM1 (monosialoganglioside) et MET C18:1 (10/1/1 mol/mol/mol) sont dissous dans du chloroforme méthanol (1:1 v/v) et ensuite évaporés sous azote. Le film lipidique est solubilisé dans 200 µl de PBS contenant 4µM d'octylglucoside. Cette suspension est ensuite dialysée contre du PBS pendant 6 heures. Les membranes de type BRL utilisées ont une limite d'exclusion de 12000-14000 KDa et la dialyse se fait à pH 7,4.

### TRAITEMENT DES TUMEURS

Les liposomes (4 µl contenant 40 µg de MET C18:1) sont injectés 72 heures après l'inoculation des cellules C6 dans le cerveau dans le lieu de la tumeur à l'aide d'un appareil stéréotaxique.

### AUTOPSIE ET HISTOCHIMIE

Les rats sont sacrifiés 8 jours après le traitement par injection sub-léthale de pentobarbital, perfusés avec du formol (4%) et le cerveau p levé est post-fixé dans du formol (4%) et ensuite dans de l'isobutanol et inclus dans de la paraffine. Des coupes de 10 µm sont réalisées à l'aide d'un microtome et après déparaffinage dans du toluène, les glioblastomes sont détectés par coloration au crésyl violet (0,1 % dans 0,1 M d'acétate d'éthyl et 0,1 M d'acétate de sodium) et soumises à un analyseur d'images (IBAS, KONTRON, SAMBA ALCATEL).

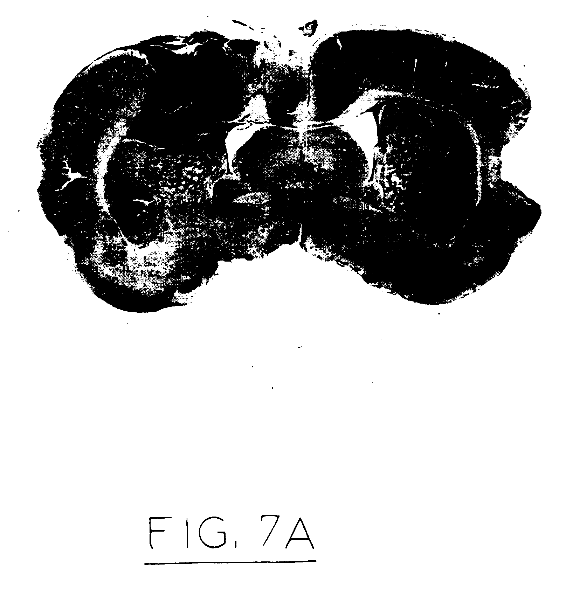

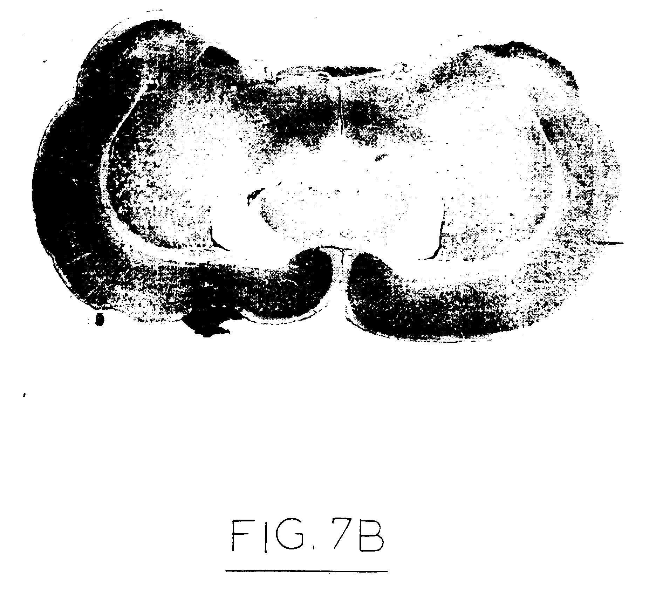

La figure 7 montre que l'injection du MET C18:1 (40 µg) dans les glioblastomes obtenus dans le cortex de rat, provoque une diminution significative de la taille des tumeurs.

La figure 7A montre le résultat obtenu après traitement par des liposomes contenant du PC et du GMI.

La figure 7B montre le résultat obtenu après traitement par des liposomes contenant du PC, du GMI et du 7β -OH-CH-3-oléate (40 µg).

### IMAGERIE PAR RESONANCE MAGNETIQUE NUCLEAIRE

Les images de cerveaux de rats sont réalisées avec un spectromètre Bruker MSL 200 équipé d'un aimant de large ouverture : 7,8 cm. Le champ magnétique de 4,7 teslas correspond à une fréquence de résonance de proton de 200 MHz. Les gradients de champs utilisés permettent l'acquisition d'images ayant une épaisseur de coupe de 1 mm à mi-hauteur et un champ de vue de 4 cm, soit une résolution latérale de 0,15 mm pour des images 256x256. Les coupes réalisées sont frontales.

L'animal est anesthésié par injection intramusculaire de kétamine (36 mg/kg) et de xylazine (5,5 mg/kg). Il est fixé sur un support permettant un positionnement reproductible et introduit dans une antenne de 40 mm de diamètre utile.

Afin de distinguer les différents tissus rencontrés et les lésions (oedème, gliose, tumeur) provoquées et de les caractériser par leur temps de relaxation longitudinale T1 et transversale T2, différentes séquences d'acquisition sont utilisées.

Dans un premier temps, les séquences d'imagerie rapide sont employées pour le positionnement correct des coupes, la localisation des lésions et la détermination du contraste observé (T1, T2). Des images pondérées en T1 sont obtenues par l'emploi de la séquence GEFI (écho de gradient) avec un temps d'écho T_{E} = 14 ms, un temps de récupération Tᵣ = 175 ms et un angle de basculement de 30°. Des images pondérées en T2 sont réalisées avec la séquence RARE (trains d'échos de spin différemment codés en phase) avec T_{E} = 30 ms, et Tᵣ = 3000 ms et un facteur RARE de 8. Ces images GEFI et RARE sont réalisées sur plusieurs coupes frontales en quelques minutes (5 à 10 minutes en tout). Seize coupes jointives permettent d'étudier la totalité du cerveau. Pour le cas des tumeurs, du Gadolinium (70 µmoles dans 9 % de NaCI) est injecté comme produit de contraste.

L'analyse quantitative des lésions ainsi observées nécessite l'acquisition d'images avec des séquences d'échos de spin à plusieurs échos et temps de récupération.

Une première série d'images est réalisée avec la séquence MSME (multislices-multi-écho). Les paramètres d'acquisition sont : T_{E} = 30 ms, Tᵣ = 2646 ms. Six échos sont enregistrés. Afin d'améliorer le rapport signal à bruit, deux accumulations sont effectuées. Le temps d'acquisition est de 23 minutes. La densité de proton et le paramètre de relaxation transversale (fonction de T2 et du coefficient de diffusion) sont calculés à l'aide d'un modèle monoexponentiel en chaque point de l'image. Leurs distributions (moyenne et écart-type) sont déterminées en différentes régions d'intérêts.

La mesure des paramètres de relaxation longitudinale nécessite l'acquisition d'images avec un taux de saturation différent de celui obtenu précedemment. La séquence MSME est alors utilisée avec un temps de récupération de 783 ms. Deux échos sont enregistrés. Le temps d'acquisition correspondant à deux accumulations est de 7 minutes. Le temps de relaxation T1 est calculé en chaque point à partir du rapport des images acquises avec les deux temps de récupération, selon un modèle d'évolution prenant en compte un seul temps de relaxation. La distribution des T1 est déterminée sur les mêmes régions d'intérêt.

Le suivi au cours du temps d'un même glioblastome induit dans le cortex d'un rat, par imagerie RMN, confirme la régression de la tumeur lors d'un traitement par le 7β -OH-cholestérol-3-oléate.

## Revendications

1. Composition pharmaceutique, caractérisée en ce qu'elle comporte au moins un mono ester en C3 de 7 β-hydroxycholestérol choisi dans le groupe comprenant le palmitate, l'oléate, l'héxénoate, le décénoate et l'arachidonate, sous forme de liposomes, et/ou associé avec des phospholipides et/ou des gangliosides, et un support acceptable pour son administration.

2. Composition pharmaceutique, caractérisée en ce qu'elle comporte au moins un éther en C-3-OH du 7 β-hydroxycholestérol choisi dans le groupe comprenant les éthers d'acide palmitique, oléique, hexénoïque, décénoïque et arachidonique, et un support acceptable pour son administration.

3. Composition selon la revendication 2, caractérisée en ce que l'éther du 7 β-hydroxycholestérol est incorporé dans des liposomes et/ou associé à des phospholipides et/ou des gangliosides.

4. Utilisation d'une composition selon l'une des revendications 1 à 3 pour la préparation de médicaments ayant une activité cytotoxique, utile notamment dans le traitement de maladies mettant en jeu des cellules à haut potentiel de prolifération.

5. Utilisation selon la revendication 4 d'une composition selon l'une des revendications 1 à 3, caractérisée en ce que le médicament est actif en tant qu'agent antitumoral.

6. Utilisation selon l'une des revendications 4 ou 5 d'une composition pharmaceutique selon l'une des revendications 1 à 3, caractérisée en ce que le médicament est destiné au traitement de tumeurs du système nerveux, en particulier de glioblastomes.

7. Utilisation selon la revendication 4 d'une composition pharmaceutique selon l'une des revendications 1 à 3, caractérisée en ce que le médicament est destiné au traitement de glioses réactionnelles.

## Claims

1. Pharmaceutical composition, characterized in that it contains at least one C3 monoester of 7β-hydroxycholesterol chosen from the group comprising the palmitate, oleate, hexenoate, decenoate and arachidonate, in the form of liposomes, and/or combined with phospholipids and/or gangliosides, and a vehicle acceptable for its administration.

2. Pharmaceutical composition, characterized in that it contains at least one C3-OH ether of 7β-hydroxycholesterol chosen from the group comprising the ethers of palmitic, oleic, hexenoic, decenoic and arachidonic acid, and a vehicle acceptable for its administration.

3. Composition according to Claim 2, characterized in that the ether of 7β-hydroxycholesterol is incorporated into liposomes and/or combined with phospholipids and/or gangliosides.

4. Use of a composition according to one of Claims 1 to 3 for the preparation of drugs having a cytotoxic activity, useful particularly in the treatment of diseases involving cells having a high proliferative potential.

5. Use according to Claim 4 of a composition according to one of Claims 1 to 3, characteri-zed in that the drug is active as an antitumoral agent.

6. Use according to either of Claims 4 and 5 of a pharmaceutical composition according to one of Claims 1 to 3, characterized in that the drug is intended for the treatment of tumours of the nervous system, in particular of glioblastomas.

7. Use according to Claim 4 of a pharmaceutical composition according to one of Claims 1 to 3, characterized in that the drug is intended for the treatment of reactional glioses.

## Patentansprüche

1. Pharmazeutische Zusammensetzung, dadurch charakterisiert, daß sie mindestens einen C3-Monoester von 7β-Hydroxycholesterin umfaßt, der ausgewählt wird aus der den Palmitinsäure-, den Ölsäure-, den Hexensäure-, den Decensäure- und den Arachidonsäureester umfassenden Gruppe, in Form von Liposomen und/oder in Kombination mit Phospholipiden und/oder Gangliosiden, und einen für die Verabreichung verträglichen Träger.

2. Pharmazeutische Zusammensetzung, dadurch charakterisiert, daß sie mindestens einen C3-OH-Ether von 7β-Hydroxycholesterin umfaßt, der ausgewählt wird aus der die Ether von Palmitinsäure, Ölsäure, Hexensäure, Decensäure und Arachidonsäure umfassenden Gruppe, und einen für die Verabreichung verträglichen Träger.

3. Zusammensetzung nach Anspruch 2, dadurch charakterisiert, daß der Ether von 7β-Hydroxycholesterin in Liposomen enthalten ist und/oder in Kombination mit Phospholipiden und/oder Gangliosiden vorliegt.

4. Verwendung einer Zusammensetzung nach einem der Ansprüche 1 bis 3 zur Herstellung von Medikamenten mit einer cytotoxischen Aktivität, die insbesondere für die Behandlung von Erkrankungen nützlich sind, bei denen Zellen mit hohem Proliferatinspotential eine Rolle spielen.

5. Verwendung nach Anspruch 4 einer Zusammensetzung nach einem der Ansprüche 1 bis 3, dadurch charakterisiert, daß das Medikament als Antitumormittel wirksam ist.

6. Verwendung nach einem der Ansprüche 4 oder 5 einer pharmazeutischen Zusammensetzung nach einem der Ansprüche 1 bis 3, dadurch charakterisiert, daß das Medikament zur Behandlung von Tumoren des Nervensystems bestimmt ist, insbesondere von Glioblastomen.

7. Verwendung nach Anspruch 4 einer Zusammensetzung nach einem der Ansprüche 1 bis 3, dadurch charakterisiert, daß das Medikament zur Behandlung von reaktiven Gliosen bestimmt ist.
